# EUROPEAN PATENT SPECIFICATION

(11) **EP 0 200 750 B1**
(45) Date of publication and mention of the grant of the patent: **07.01.1993**
(21) Application number: 85905256.5
(22) Date of filing: 02.10.1985
(51) Int. Cl.: A61K 49/00, A61B 5/05, A61B 6/00, G01N 24/00, C07F 15/00, C07F 15/02

(54) **DIESTER-DTPA-PARAMAGNETIC CONTRAST AGENTS FOR MRI IMAGING, APPARATUS AND METHODS**
DIESTERPARAMAGNETISCHE KONTRASTMITTEL FÜR DIE BILDFORMUNG DURCH MAGNETISCHE RESONANZ, APPARAT UND VERFAHREN
AGENTS DE CONTRASTE PARAMAGNETIQUE-DIESTER-DTPA POUR FORMATION D'IMAGES PAR RESONANCE MAGNETIQUE, APPAREIL ET PROCEDES

(30) Priority: 04.10.1984 US 657676
(43) Date of publication of application: 12.11.1986
(73) Proprietor: NYCOMED SALUTAR, INC., Sunnyvale, California 94086 (US)
(72) Inventor: QUAY, Steven, C., Menlo Park, CA 94305 (US)
(74) Representative: Cockbain, Julian, Dr.
(86) International application number: PCT/US85/01915
(87) International publication number: WO 86/02005

(56) References cited:
- EP-A- 0 071 564
- EP-A- 0 133 603
- Chemical Abstracts, Vol. 101, No. 24, issued 1984, December 10, A.G. Schering, Belgian Patent No. BE 898-708, 16 May 1984, Diagnostic composition containing complexes, Abstract No. 101: 216407q
- Chemical Abstracts, Vol. 81, No. 13, issued 1974, September 30, W.H. Mueller, Synthesis of the ethyl esters of nitrilotriacetic acid..., Abstract No.77423v.
- Chemical Abstracts, Vol. 90, No. 25, issued 1979, June 18, R.A. Guilmette, et al., Synthesis and therapeutic testing..., Abstract No. 90: 203450c

## Description

This invention relates to magnetic resonance imaging (MRI) contrast agents, and more particularly to contrast agents comprising paramagnetic metal complexes of esters of diethylenetriaminepentaacetic acid (DTPA).

DE-A-3129906 (Schering/Gries et al) teaches the incorporation of paramagnetic metals into DTPA to form chelates useful as contrast agents in magnetic resonance imaging. The contrast agent DTPA-Gd(III) as taught by Schering is insoluble in water and requires the addition of cations "C+" (amines such as glucamine and N-methylglucamine as shown below. The charge balance of the Schering's DTPA-Gd(III) is: 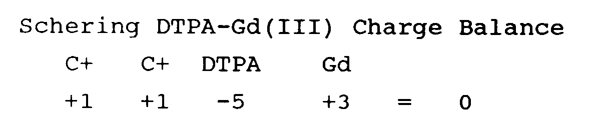
The resulting contrast agent has three ion particles in solution for each paramagnetic atom (PM)- a particle to PM ratio of 3:1. A paramagnetic metal with a valence of two, such as Mn, would require an additional glucamine ion: 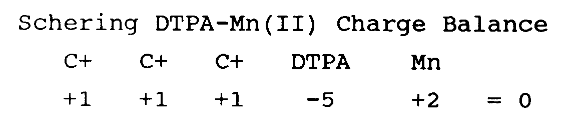
raising the particle to PM ratio to 4:1.

These contrast agents raise the in vivo ion concentration and disturb the local osmolarity balance. The osmolarity is normally regulated at about 300 milliosmols per liter. Increasing the osmolarity with injected ions causes water to collect within the unbalanced region which dilutes the ion concentration.

The present invention is based on the discovery that by esterifying one or more of the carboxy groups of the chelating agent DTPA the paramagnetic metal complexes of the modified chelating agents (Ester-DTPA) are improved MRI contrast agents in that they have high stability, low toxicity and are physiologically tolerable. The improved Ester-DTPA-PM contrast agents of the invention may be provided in pharmacological form having a low osmolarity and may be organ selective.

Thus in one aspect the present invention provides a physiologically tolerable complex of a polyvalent paramagnetic metal ion and a C₁₋₁₆ or C₁₈ linear alkyl ester of diethylenetriaminepentaacetic acid.

In a further aspect the invention also provides a magnetic resonance imaging contrast medium composition comprising a physiologically tolerable complex of paramagnetic metal ion and a C₁₋₁₆ linear alkyl ester of DTPA together with a pharmaceutically acceptable carrier.

In a yet further aspect the invention provides the use of a physiologically tolerable complex of a paramagnetic metal ion and a C₁₋₁₆ linear alkyl ester of DTPA for the manufacture of a magnetic resonance imaging contrast medium composition.

The chemically stable, physiologically tolerable contrast agents may be provided in a pharmacological state for in vivo use during diagnostic magnetic resonance (MR) imaging. The contrast agent enhances the MR image of a subject within the MRI scanning magnetic field. A paramagnetic metal ion PM(+Z) having an atomic charge of Z locally affects the MRI scanning magnetic field to reduce the T₁ relaxation time of local protons within the subject. The contrast agent contains a triamine chelator securely polar bonded around the PM(+Z) ion at a plurality of coordination points, and has the form:

(HOOCCH₂)₂NCH₂CH₂N(CH₂COOH)CH₂CH₂N(CH₂COOH)₂

in which at least one, preferably two, of the carboxy groups are replaced by ester groups of the form -COO(CH₂)ₙH where n is an integer of 1 to 16, e.g. to produce a chelating agent Ester-DTPA having the form (HOOCCH₂)(H(CH₂)ₙOOCCH₂)NCH₂CH₂N(CH₂COOH)CH₂CH₂N(CH₂COOH) (CH₂-COO(CH₂)ₙH). This serves to chemically isolate the PM(+Z) ion from the in vivo environment.

The Ester-DTPA may for example be a homo-diester or a heterodiester. The Ester-DTPA-PM contrast agent may be dispersed in a pharmaceutically acceptable vehicle means (carrier) such as water. The carbon-hydrogen portion of the ester compound becomes associated with water of hydration which increases the paramagnetic strength of the contrast agent. The PM ion may have a valence of +3 and produce a contrast agent molecule of zero net charge. The PM ion may have a valence of +2 and require an inert cation IN having an atomic charge to produce a molecule with a zero net charge. The paramagnetic metal ion PM(+Z) may be the ion of one of the Transition Elements 24-29 or the Lanthanide Elements 57-71.

The invention will now be further illustrated with reference to the accompanying drawings, in which:
Figure 1A is a diagram showing the chelate structure and water of hydration of a Diester-DTPA-PM(Z) contrast agent in which Z=+3;
Figure 1B is a diagram showing the chemical structure of the Diester-DTPA-PM contrast agent of Figure 1A;
Figure 1C is a diagram showing the chemical structure of a Dibutylester-DTPA-PM(Z) contrast agent in which Z=+2;
Figure 2 is a diagram showing the anhydride+methanol production of Dimethylester-DTPA-PM(Z) in which Z=+3;
Figure 3 is a diagram showing the anhydride+methanol production of Dibutylester-DTPA-PM(Z) in which Z=+2; and
Figure 4 is a chart showing the organ selectivity of homologs of Diester-DTPA-PM paramagnetic contrast agents.

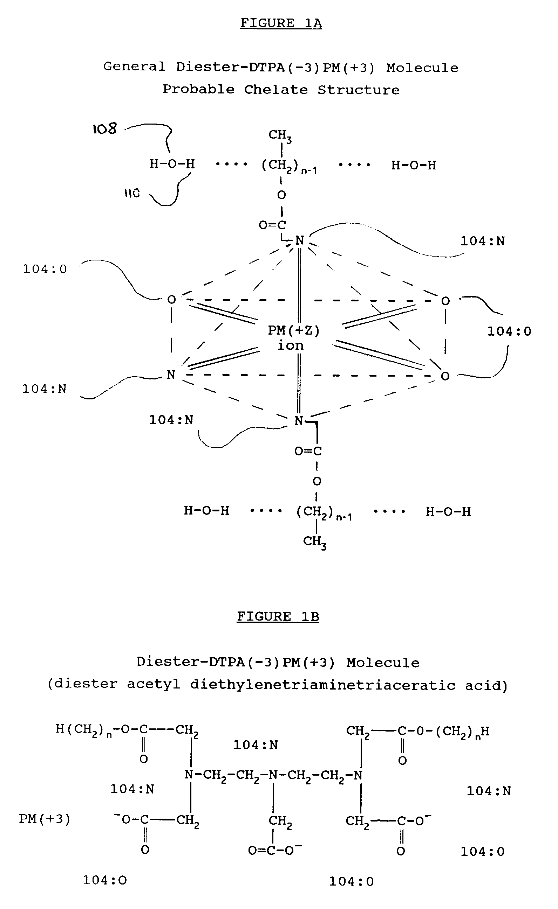

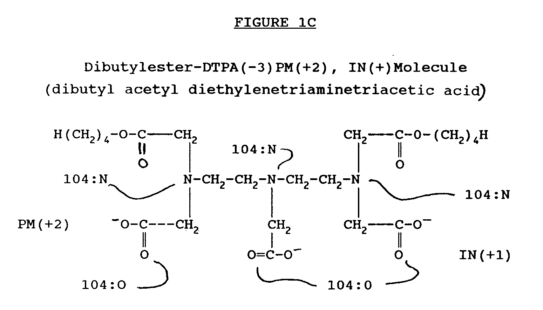

The paramagnetic contrast agents of the present invention include ester homologs of DTPA-PM chelates having the general chemical name diester acetyl - diethlenetriaminetriacetic acid (or Diester-DTPA). The probable physical chelation structure of Diester-DTPA-PM is a classic octahedron (8 faces, 6 apexes) as shown in Figure 1A. The Diester-DTPA homologs are strong chelators with six polar bond coordination points 104 (three nitrogen points 104:N and three oxygen points 104:0) which enclose the paramagnetic ion PM(Z) on all sides.

Diester-DTPA-PM has the general chemical structure shown in Figure 1B. The homologs of Diester-DTPA-PM(Z) have similar structures with a specific number "n" of carbons in the carbon-hydrogen portion of the ester group. The number of carbons in the alkylene chain between the -COO- active group and the terminal methyl -CH₃, is "n-1". Two of the original five DTPA carboxy groups have become ester groups "E". In general:
Diester-DTPA-PM = 2E-DTPA-PM where E is a general ester group of the form: 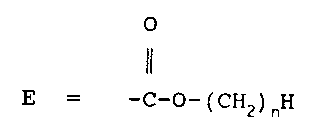
and PM is a paramagnetic metal ion. The elimination of two carboxy groups reduces the ion charge of the DTPA chelator from five to three.

Paramagnetic ions having a valence of Z=+3 as shown in Figure 1A and 1B, produce a diester contrast agent of the general form:
Diester-DTPA-PM(+3) = 2E-DTPA-PM(+3). This type III contrast agent has a zero net charge as tabulated below: 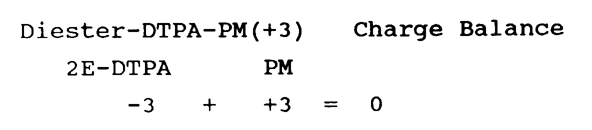
The particle (osmolarity) to paramagnetic (molar relaxivity) ratio for Diester-DTPA-PM(+3) type contrast agents (Z=3) is 1:1. The Diester-DTPA-PM(Z) contrast agents formed around paramagnetic metal ions having a charge of +3 can be prepared in highly concentrated solutions while retaining isotonicity with body fluids. The Schering DTPA-PM(+3) contrast agent has a particle to paramagnetic metal ion ratio of 3:1, and can only be made in isotonic solutions at substantially lower concentrations. Therefore, greater volumes of the Schering DTPA-PM(+3) contrast agent need be injected into animals or humans to obtain the same paramagnetic effect.

Paramagnetic metal ions having a valence of Z=2, produce ester contrast agents of the general form:
Diester-DTPA-PM(+2),IN = 2E-DTPA-PM(+2),IN where IN is a suitable inert ion, such as a simple mineral salt cation (Na⁺, Li⁺, etc.) or an organic ion such as methyl glucamine or N-methyl glucamine, having a charge of plus one (see Figure 1C). This type II contrast agent also has a zero net charge as tabulated below: 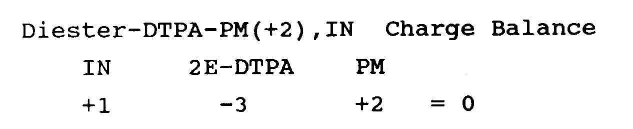
The particle to paramagnetic metal ion ratio for the Diester-DTPA-PM(+2),IN contrast agents is 2:1, producing a low osmolarity impact.

The above Diester-DTPA-PM Type III and Type II contrast agents have a higher paramagnetic effect than the Schering DTPA-PM contrast agent. For example, Methylester-DTPA-Gd(III) requires a concentration of only about 1.91 mM to produce a T₁ relaxation time of 67 msec (10 MHz field strength, using an RADX). The concentration of Schering's DTPA-Gd(III) required to produce a similar result is about 3.16 mM. Methylester-DTPA-Gd(III) has about twice the paramagnetism of Schering's DTPA-Gd(III); and Methylester-DTPA-Fe(III) has about 1.3 times the paramagnetism of Schering's DTPA-Fe(III). Possibly the water of hydration 108 (see Figure 1A) which collects around the ester CH₂ chains offers a reliable source of protons 110 for resonanting with the applied MRI fields. Protons 110 have a high probability of being present within the local magnetic field of the PM ions. These protons form a class of protons for MRI imaging which is distinct from random in vivo protons. The prolonged association time of bound water 108, and the close proximity of protons 110 to the PM ion, establishes a definite and distinct T₁ relaxation time which is longer than the T₁ for random protons. As a result, protons 110 provided by the water of hydration appear at a higher intensity in the MRI image.

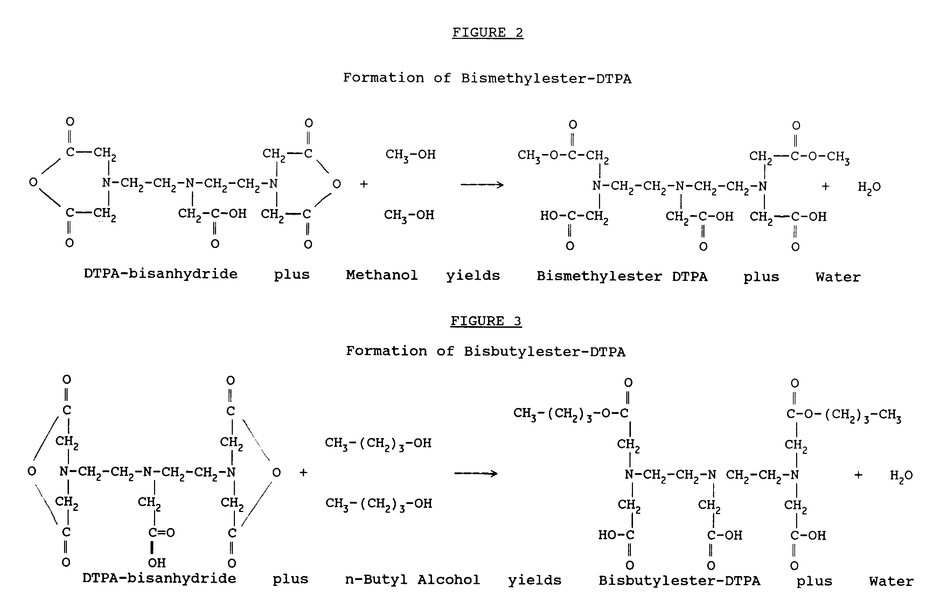

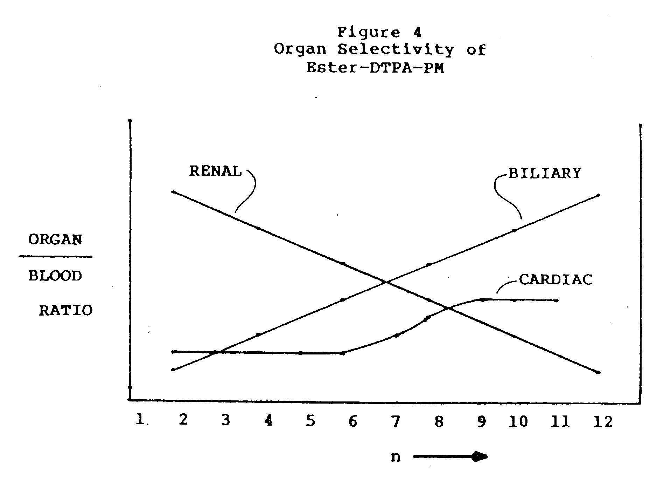

A general anhydride-diester method, as illustrated in Figures 2 and 3, is suitable for making each homolog of the ester family of Ester-DTPA-PM contrast agents. In the example below, the paramagnetic ion is provided by Fe(III)Cl₃ for chelation into the dimethyl ester (n=1). However, other paramagnetic ions in other forms may be employed for chelation into other ester homologs.
- Step 1): FORMATION of Ester-DTPA (see Figure 2) Mix 1-5 grams dianhydride DTPA (obtained from Sigma Chemical Co., St Louis, Missouri, USA) into 50-150 mL of pure methanol. The alcohol forms both the reactant and the solvent for the DTPA anhydride. Precise ratios of alcohol/DTPA are not required so long as excess alcohol is provided.
- Step 2): HEAT the solution for several hours (overnight) at reflux temperature to produce the ester derivative Dimethylester-DTPA (n=1) plus water. Higher homologs of Diester-DTPA may be formed using the corresponding higher homolog of the alcohol for the solvent-reactant. Chloroform may be used as the solvent for higher homologs. Formation of the Dibutylester-DTPA (n=4) diester homolog is shown in Figure 3.
- Step 3): REMOVE the excess alcohol by vacuum rotary evaporation leaving a Diester-DTPA crystal residue.
- Step 4): MIX the Diester-DTPA residue in an aqueous FeCl₃ solution of stochiometric proportions to form Diester-DTPA-Fe³⁺ plus 3HCl. Type II metals will require an inert cation (IN) which may be added to the solution at this point.
- Step 5): REMOVE the HCl
A) by evaporation using a rotary evaporator.
B) by neutralization using NaOH or NH₄OH.
C) by chromatography using a silica gel column.
- Step 6): REMOVE the water by vacuum-freezing to form a highly stable form of Diester-DTPA-PM.
- Step 7): DISPERSE the Ester-DTPA-PM in suitable vehicle to provide a pharmacological form.
Water is a suitable vehicle for dissolving the lower homologs of Diester-DTPA-PM (n less than 10). Higher homologs are hydrophobic and form an emulsion with water. These higher homologs have the same density as water and therefore do not settle out. The isodense character of the homologs of Diester-DTPA-PM permits a wide range of water:homolog ratios.

Thus in a further aspect the invention provides a process for the manufacture of Diester-DTPA-PM complexes comprising heating an anhydride of diethylaminetriaminepentaacetic acid with a C₁₋₁₆ n-alkan-1-ol and subsequently contacting the ester chelating agent thereby produced with a solution containing a paramagnetic metal cation.

The family of Ester-DTPA-PM contrast agents include the homo-diester (n=n') structure and the hetero-diester (n not equal to n') structure.

| Name of Ester-DTPA-PM | n,n' | Properties of Interest |
|---|---|---|
| Bismethylester-DTPA-PM | 1,1 | Excellent renal and blood-brain barrier contrast agent. |
| Bisethylester-DTPA-PM | 2,2 | |
| Bispropylester-DTPA-PM | 3,3 | |
| Bisbutylester-DTPA-PM | 4,4 | |
| Bispentylester-DTPA-PM | 5,5 | Demonstrates renal and hepatobiliary imaging. |
| Bishexylester-DTPA-PM | 6,6 | |
| Bisheptylester-DTPA-PM | 7,7 | |
| Bisoctylester-DTPA-PM | 8,8 | Also shows cardiac imaging of infarctions and ischemic lesions. |
| Bisnonylester-DTPA-PM | 9,9 | |
| Bisdecylester-DTPA-PM | 10,10 | |
| to | 16,16 | |

The hetero-diesters may have one short alkylene chain (n=1 or more), and one long alkylene chain (n=16 less). A single long hydrophobic chain, together with the charged DTPA residue, renders the chelate an isosteric substitute for fatty acids and produces substantial tissue levels of the chelate in those organs which have efficient fatty acid uptake systems, such as the myocardium.

Contrast agents according to the invention introduced into the systemic vasculature are immediately distributed through the circulatory system for imaging. The distribution to organs is based on relative blood flow. Organs such as kidney, brain, liver and heart receive substantial blood flow and therefore provide selective images which are correspondingly enhanced.

The higher homologs of Ester-DTPA-PM tend to be less polar and to bind to serum proteins prolonging their circulation time. They also tend to be extracted from circulation by the liver and excreted in the hepatobiliary system. These homologs are liver selective and suitable for imaging the liver and hepatobiliary (gall bladder) system.

The lower homologs tend to be more polar and remain in solution longer. They are eventually absorbed by the kidney. These homologs are kidney selective and suitable for imaging the kidney, ureter and bladder.

The higher homologs are fatty acids analogs and are thus extracted by the heart along with the regular fatty acids. These homologs (n=7 and greater) are cardiac selective and suitable for imaging the cardiac system and cardiac related functions.

The Diester-DTPA-PM contrast agents in stable powder state have an indefinite shelf life, and this is the preferred state for shipping and storage. The contrast agent in water solution (or other solvent) is packaged in small storage vials, and frozen under a vacuum. The low pressure sublimates the solvent, leaving crystals of the contrast agent. The vial is sealed to prevent entry of external contaminants, and to preserve the internal vacuum. The resulting freeze-dried, vacuum sealed powder, is highly stable and free from environmental degradation effects.

Prior to injection, the stable-powdered contrast agent may be raised to the pharmacological state by the addition of a suitable solvent such as water, serum, albumin solutions, or saline. A typical injectable composition contains about 10mg human serum albumin (1 percent USP Parke-Davis) and from 10 to 500 micrograms of Diester-DTPA-PM material per millilitre of 0.01 M phosphate buffer (pH 7.5) containing 0.9 percent NaCl. The pH of the aqueous solutions may range between 5-9, preferably between 6-8. The storage vial may have twin compartments containing the desired amounts of powdered Diester-DTPA-PM and solvent for a single application. When the seal between the compartments is broken, the Diester-DTPA-PM goes into solution at the desired concentration for immediate use. The Diester-DTPA-PM solution mixes readily with the in vivo fluids.

The paramagnetic species PM may be any paramagnetic element, molecule, ion or compound having a combined valence of "Z". Paramagnetic material PM includes at least one of the following elements:

| Ions of Transition Elements | | | | | | |
|---|---|---|---|---|---|---|
| Cr | (III) | 24 | (Chromium) | Co(II) | 27 | (Cobalt) |
| Mn | (III) | 25 | (Manganese) | Ni(II) | 28 | (Nickel) |
| Fe | (III) | 26 | (Iron) | Cu(III) | 29 | (Copper) |
| Fe | (II) | 26 | (Iron) | Cu(II) | 29 | (Copper) |

| Ions of Lanthanide Elements | | | | | | |
|---|---|---|---|---|---|---|
| La | (III) | 57 | (Lanthanum) | Gd(III) | 64 | (Gadolinium) |
| Ce | (III) | 58 | (Cerium) | Tb(III) | 65 | (Terbium) |
| Pr | (III) | 59 | (Praseodymium) | Dy(III) | 66 | (Dysprosium) |
| Nd | (III) | 60 | (Neodymium) | Ho(III) | 67 | (Holmium) |
| Pm | (III) | 61 | (Promethium) | Er(III) | 68 | (Erbium) |
| Sm | (III) | 62 | (Samarium) | Tm(III) | 69 | (Thulium) |
| Eu | (III) | 63 | (Europium) | Yb(III) | 70 | (Ytterbium) |
| | | | | Lb(III) | 71 | (Lutetium) |

Gd has the highest paramagnetic property; but is costly and is highly toxic in the free state. Placing the Gd within the chelator produces a physiologically tolerable form of Gd but also reduces the paramagnetic effect of the Gd. The chelate structure tends to shield the paramagnetic ions and prevents close proximity to local protons. Fe and Mn have a high paramagnetic property and excellent physiological tolerance. Both of these paramagnetic ions are normally present in the physiological environment.

A magnetic resonance imaging system and its use are discussed in WO-A-86/02005 and discussed further in Scientific American, May 1982, pages 78-88.

## Claims

1. A physiologically tolerable complex of a polyvalent paramagnetic metal ion and a C₁₋₁₆- linear alkyl ester of diethylenetriaminepentaacetic acid.

2. A complex as claimed in claim 1 wherein said metal is a lanthanide.

3. A complex as claimed in claim 1 wherein said metal is iron.

4. A complex as claimed in claim 1 wherein said metal is manganese.

5. A complex as claimed in claim 1 wherein said metal is gadolinium.

6. A complex according to any one of claims 1 to 5 wherein said metal is complexed by the residue of a said ester of formula
R-OCOCH₂N(CH₂COOH)CH₂CH₂N(CH₂COOH)CH₂CH₂N(CH₂COOH)CH₂COOR
(where each R is a C₁₋₁₆ linear alkyl group).

7. A complex as claimed in any one of claims 1 to 6 wherein said ester is a homodiester.

8. A complex as claimed in any one of claims 1 to 6 wherein said ester is a heterodiester.

9. A complex as claimed in any one of claims 1 to 6 wherein said ester is a bismethylester of diethylenetriaminepentaacetic acid.

10. A complex as claimed in any one of claims 1 to 6 wherein said ester is a bisbutylester of diethylenetriaminepentaacetic acid.

11. A complex as claimed in any one of claims 1 to 10 for use as an MRI contrast agent.

12. A magnetic resonance imaging contrast medium composition comprising a complex as claimed in any one of claims 1 to 11 together with a pharmaceutically acceptable carrier.

13. The use of a complex as claimed in any one of claims 1 to 11 for the manufacture of a magnetic resonance imaging contrast medium composition.

14. A process for the manufacture of a complex as claimed in any one of claims 1 to 11 comprising heating an anhydride of diethylenetriaminepentaacetic acid with a C₁₋₁₆ n-alkan-1-ol and subsequently contacting the ester chelating agent thereby produced with a solution containing a paramagnetic metal cation.

## Patentansprüche

1. Physiologisch verträglicher Komplex eines polyvalenten paramagnetischen Metallions und eines linearen C₁-₁₆-Alkylesters der Diethylentriaminpentaessigsäure.

2. Komplex nach Anspruch 1, worin das Metall ein Lanthanid ist.

3. Komplex nach Anspruch 1, worin das Metall Eisen ist.

4. Komplex nach Anspruch 1, worin das Metall Mangan ist.

5. Komplex nach Anspruch 1, worin das Metall Gadolinium ist.

6. Komplex nach einem der Ansprüche 1 bis 5, worin das Metall über den Rest eines Esters der Formel
R-OCOCH₂N(CH₂COOH)CH₂CH₂N(CH₂COOH)CH₂CH₂N(CH₂COOH)CH₂COOR
(worin jedes R für eine lineare C₁-C₁₆-Alkylgruppe steht), komplexiert ist.

7. Komplex nach einem der Ansprüche 1 bis 6, worin der Ester ein Homodiester ist.

8. Komplex nach einem der Ansprüche 1 bis 6, worin der Ester ein Heterodiester ist.

9. Komplex nach einem der Ansprüche 1 bis 6, worin der Ester ein Bismethylester der Diethylentriaminpentaessigsäure ist.

10. Komplex nach einem der Ansprüche 1 bis 6, worin der Ester ein Bisbutylester der Diethylentriaminpentaessigsäure ist.

11. Komplex nach einem der Ansprüche 1 bis 10 zur Verwendung als MRI-Kontrastmittel.

12. Zusammensetzung für ein Magnetic Resonance Imaging Kontrastmedium, umfassend einen Komplex nach einem der Ansprüche 1 bis 11 zusammen mit einem pharmazeutisch verträglichen Träger.

13. Verwendung eines Komplexes nach einem der Ansprüche 1 bis 11 zur Herstellung einer Zusammensetzung für ein Magnetic Resonance Imaging Kontrastmedium.

14. Verfahren zur Herstellung eines Komplexes nach einem der Ansprüche 1 bis 11, wobei man ein Anhydrid der Diethylentriaminpentaessigsäure mit einem C₁-C₁₆-n-Alkan-1-ol erhitzt und anschließend den dabei anfallenden chelatbildenden Ester mit einer Lösung in Kontakt bringt, welche ein Kation eines paramagnetischen Metalls enthält.

## Revendications

1. Complexe physiologiquement acceptable d'un ton de métal paramagnétique polyvalent et d'un ester d'alkyle linéaire en C₁₋₁₆ de l'acide diéthylènetriaminepentaacétique.

2. Complexe selon la revendication 1, dans lequel ledit métal est un lanthanide.

3. Complexe selon la revendication 1, dans lequel ledit métal est le fer.

4. Complexe selon la revendication 1, dans lequel ledit métal est le manganèse.

5. Complexe selon la revendication 1, dans lequel ledit métal est le gadolinium.

6. Complexe selon l'une quelconque des revendications 1 à 5, dans lequel ledit métal est complexé par le reste d'undit ester de formule
R-OCOCH₂N(CH₂COOH)CH₂CH₂N(CH₂COOH)CH₂CH₂N(CH₂COOH)CH₂COOR
(dans laquelle chaque R est un groupe alkyle linéaire en C₁₋₁₆).

7. Complexe selon l'une quelconque des revendications 1 à 6, dans lequel ledit ester est un homodiester.

8. Complexe selon l'une quelconque des revendications 1 à 6, dans lequel ledit ester est un hétérodiester.

9. Complexe selon l'une quelconque des revendications 1 à 6, dans lequel ledit ester est un bisester méthylique de l'acide diéthylènetriaminepentaacétique.

10. Complexe selon l'une quelconque des revendications 1 à 6, dans lequel ledit ester est un bisester butylique de l'acide diéthylènetriaminepentaacétique.

11. Complexe selon l'une quelconque des revendications 1 à 10 destiné à être utilisé comme agent de contraste en IRM.

12. Composition de milieu de contraste pour l'imagerie par résonance magnétique comprenant un complexe selon l'une quelconque des revendications 1 à 11 avec un véhicule pharmaceutiquement acceptable.

13. Utilisation d'un complexe selon l'une quelconque des revendications 1 à 11 pour la préparation d'une composition d'agent de contraste pour l'imagerie par résonance magnétique.

14. Procédé pour la préparation d'un complexe selon l'une quelconque des revendications 1 à 11 comprenant le chauffage d'un anhydride de l'acide diéthylènetriaminepentaacétique avec un n-alcanol-1 en C₁₋₁₆, puis le contact de l'ester chélateur ainsi produit avec une solution contenant un cation de métal paramagnétique.
